(19) Europäisches Patentamt
European Patent Office
Office européen des brevets

(11) Veröffentlichungsnummer: **0 089 587**
**B1**

(12) # EUROPÄISCHE PATENTSCHRIFT

(45) Veröffentlichungstag der Patentschrift: **29.10.86**

(51) Int. Cl.⁴: **B 01 J 23/62**, C 07 C 67/39, C 07 C 67/54

(21) Anmeldenummer: **83102454.2**

(22) Anmeldetag: **12.03.83**

(54) **Katalysator und seine Verwendung zur Herstellung von Methylmethacrylat.**

(30) Priorität: **24.03.82 DE 3210708**

(43) Veröffentlichungstag der Anmeldung: **28.09.83 Patentblatt 83/39**

(45) Bekanntmachung des Hinweises auf die Patenterteilung: **29.10.86 Patentblatt 86/44**

(84) Benannte Vertragsstaaten: **BE DE FR GB IT NL**

(56) Entgegenhaltungen:
**DE - A - 2 025 992**
**DE - A - 2 848 369**
**DE - A - 3 639 449**
**GB - A - 2 070 601**
**US - A - 3 959 354**

(73) Patentinhaber: **BASF Aktiengesellschaft, Carl-Bosch-Strasse 38, D-6700 Ludwigshafen (DE)**

(72) Erfinder: **Broecker, Franz Josef, Dr., Schwanthaler Allee 20, D-6700 Ludwigshafen (DE)**
Erfinder: **Duembgen, Gerd, Dr., Sudetenstrasse 4, D-6701 Dannstadt-Schauernheim (DE)**
Erfinder: **Fouquet, Gerd, Dr., Maxburgstrasse 2, D-6730 Neustadt (DE)**
Erfinder: **Krabetz, Richard, Dr., Unterer Waldweg 8, D-6719 Kirchheim (DE)**
Erfinder: **Merger, Franz, Dr., Max-Slevogt-Strasse 25, D-6710 Frankenthal (DE)**
Erfinder: **Nees, Friedbert, Dr., Fichtestrasse 4, D-7513 Stutensee (DE)**

EP 0 089 587 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Herstellung von Carbonsäureestern aus Aldehyden und Alkoholen in Gegenwart von Sauerstoff unter dem Einfluss von Katalysatoren ist ein seit einiger Zeit von mehreren Seiten bearbeitetes Verfahren. Für dieses Verfahren sind verschiedenartige Katalysatoren vorgeschlagen worden, von denen besonders solche, die als aktiven Bestandteil Palladium enthalten, auf Interesse gestossen sind. Die bisher bekanntgewordenen Katalysatoren dieser Art haben aber noch nicht alle Wünsche der Anwender voll befriedigen können, vor allem, soweit sie zur Herstellung von Estern α,β-ungesättigter aliphatischer Carbonsäuren, wie insbesondere Methylmethacrylat (im folgenden auch «MMA» genannt), verwendet werden.

Aus der DE-C 2 025 992 ist es bekannt, für die Umsetzung α,β-ungesättigter aliphatischer Aldehyde mit niederen einwertigen primären oder sekundären Alkoholen und molekularem Sauerstoff zu Estern α,β-ungesättigter aliphatischer Carbonsäuren als Katalysator metallisches Palladium zu verwenden, das gegebenenfalls auf einem geeigneten Träger, wie insbesondere Aluminiumoxid oder Siliciumdioxid, aufgebracht ist. Dieser Katalysator hat den Nachteil, dass er erhebliche Mengen an Nebenprodukten (Methylformiat 27 Gew.% und Formaldehyd 18 Gew.% bzg. auf Methylmethacrylat) liefert. Ausserdem werden nur geringe Umsätze oder nur geringe Selektivitäten erreicht, da man aus eingesetzten 3,58 mol Methacrolein/1 Katalysator pro h lediglich 0.11 mol Methylmethacrylat/1 Katalysator pro h erhält (vgl. dort Beispiel 4).

Die US-Patentschrift 3 639 449 beschreibt ein sehr ähnliches Verfahren zur Darstellung von Carbonsäureestern aus Aldehyden und/oder Alkoholen durch Reaktion mit Sauerstoff an Edelmetall-Katalysatoren (z.B. Palladium) bei 0 bis 300 °C. Auch hier zeigt sich die mangelhafte Brauchbarkeit eines reinen Palladiumkatalysators für die Herstellung von Methylmethacrylat: Beispiel 16, das einzige, das die Darstellung von Methylmethacrylat aus Methanol und Methacrolein an einem Katalysator 2% Pd auf Aktivkohle erläutert, gibt einen Methacrolein-Umsatz von 17,3% an mit einer Selektivität von 56,1% zu Methylmethacrylat und 40,6% zu Propylen.

Aus der DE-B- 2 848 369 ist ein Katalysator zur Herstellung von Carbonsäureestern durch Umsetzung von Aldehyden mit Alkoholen in Gegenwart von Sauerstoff bei 0 bis 200 °C bekannt, der a) Palladium, b) ein Oxid, Hydroxid, Carbonat, Nitrat oder Carbonsäuresalz von Blei, Thallium oder Quecksilber und c) ein Oxid, Hydroxid, Carbonat oder Carbonsäuresalz eines Alkali- oder Erdalkalimetalls enthält. Mit einem solchen Katalysator erhält man zwar hohe Methylacrylat-Selektivitäten (90 bis 95%), jedoch lässt die Raum-Zeit-Ausbeute, ausgedrückt als Produktivität (g MMa pro g Pd pro h), zu wünschen übrig. Dazu muss beachtet werden, dass die Zahlenangaben für die Produktivität in der Tabelle 1 der DE-AS bei niedrigem Umsatz ermittelt wurden (s. letzte Zeile der Anmerkung zur Tabelle) und daher kein reales Bild der Produktivität in Bezug auf den Gesamtumsatz geben; bezogen auf den Gesamtumsatz zu Methylmethacrylat liegt die Produktivität zwischen 2 und 10.5.

Es ist ferner gemäss GB-A- 2 070 601 bekannt, für den gattungsgemässen Katalysator Trägermaterial wie beispielsweise $Al_2O_3$ oder $TiO_2$ zu verwenden.

Es wurde nun gefunden, dass man die erwähnten Mängel mit einem Katalysator, der Palladium und Blei als aktive Bestandteile enthält, vermeiden kann, wenn die aktiven Bestandteile sich auf einem Träger befinden, der wenigstens zwei der Oxide ZnO, $Al_2O_3$, $La_2O_3$ und $TiO_2$ enthält, wobei $Al_2O_3$ und $TiO_2$ als alleinige Bestandteile ausgeschlossen sind und der Katalysator, bezogen auf sein Gesamtgewicht, nicht mehr als 0,02% an Alkali oder Erdalkali, berechnet als Metall, enthält.

Daraus ergibt sich, dass für den erfindungsgemässen Katalysator gewisse, sonst gebräuchliche Trägersubstanzen, wie Calciumcarbonat, Kieselgur und Bimsstein, wegen ihres Alkali- und/oder Erdalkaligehalts auch als untergeordnete Trägerbestandteile nicht in Betracht kommen. Das gleiche gilt für die meisten Kieselgelsorten wegen ihres erheblichen Gehalts an Alkali. Sehr bewährt haben sich Träger, die im wesentlichen aus ZnO und $Al_2O_3$, ZnO und $La_2O_3$, ZnO und $TiO_2$ oder $La_2O_3$ und $TiO_2$ bestehen. Besonders gute Ergebnisse werden mit einem Träger erzielt, der durch Calcinieren der Verbindung $Zn_6Al_2(OH)_{12}(CO_3)_3$ entstanden ist und demgemäss aus ZnO und $Al_2O_3$, offenbar in einer besonders aktiven Struktur, besteht. Diesen Träger erhält man durch gemeinsames Ausfällen von $Zn(NO_3)_2$ und $Al(NO_3)_3$ mit Soda, Abfiltrieren des gebildeten Niederschlags, Auswaschen des Alkalis, Trocknen, Calcinieren und Verpressen. Das calcinierte Produkt kann dann entweder zu Strängen verarbeitet oder zu Tabletten verpresst werden. Bei der im Beispiel 1 beschriebenen Herstellung des Trägers wird bei der Fällung ein ganz bestimmtes Zn-Al-Hydroxid-Carbonat erhalten, das durch die im Guinier-Pulverdiagramm auftretenden Linien charakterisiert wird. Die analytische Zusammensetzung entspricht der Summenfolmel

$$Zn_6\ Al_2\ (OH)_{12}\ (CO_3)_3$$

Der erfindungsgemässe Katalysator sollte eine innere Oberfläche von 10 bis 60 $m^2/g$, bestimmt nach BET, haben. Das erreicht man dadurch, dass man einen Träger zu seiner Herstellung verwendet, dessen BET-Oberfläche etwa im Bereich von 40 bis 140 $g^2/g$ liegt. Das Verfahren zur Bestimmung der sogenannten BET-Oberfläche ist erläutert in R. Haul und G. Dümbgen, Chem.-Ing.-Techn. 35, 586–589 (1963).

Der erfindungsgemässe Katalysator enthält das Palladium in metallischer Form in einer Menge von 0,1 bis 10 Gew.%, vorzugsweise 0,2 bis 5 Gew.% und besonders bevorzugt 0,5 bis 2 Gew.%, bezogen auf sein Gesamtgewicht, d.h. einschliesslich Träger. Das Blei ist in dem Katalysator als metallisches Blei oder in Form von Bleiverbindungen in einer Menge von 0,1 bis 20

Gew.%, vorzugsweise 0,2 bis 10 Gew.% und besonders bevorzugt 0,2 bis 2 Gew.%, berechnet als Metall, bezogen auf das Gesamtgewicht, vorhanden. Man kann den Katalysator in an sich bekannter Weise herstellen, beispielsweise indem man den Träger zunächst mit einer wässrigen Lösung eines Palladiumsalzes, wie Palladiumchlorid, und dann mit einer wässrigen Lösung eines Bleisalzes, wie Bleiacetat, solange behandelt, bis die Salze von dem Träger aufgenommen sind und zwischen oder nach den beiden Imprägnierungen eine Behandlung mit einem Reduktionsmittel, wie Wasserstoff oder Formaldehyd, vornimmt und den fertigen Katalysator zum Schluss trocknet.

Der erfindungsgemässe Katalysator eignet sich hervorragend zur Herstellung von Carbonsäureestern, insbesondere α,β-ungesättigten Carbonsäureestern aus entsprechenden Aldehyden und aus Alkoholen in Gegenwart von Sauerstoff. Mit besonderem Vorteil lässt er sich für die Herstellung von Methylmethacrylat aus Methacrolein, Methanol und Sauerstoff bei Temperaturen von 20 bis 100 °C verwenden. Dabei kann man in der Gasphase oder, und das wird bevorzugt, in flüssiger Phase arbeiten.

Der Einsatz des erfindungsgemässen Katalysators kann im diskontinuierlichen oder im kontinuierlichen Betrieb erfolgen. Im übrigen erfolgt die Herstellung von Methylmethacrylat unter Verwendung des erfindungsgemässen Katalysators in der bei Verwendung bekannter Katalysatoren üblichen Weise. Das Molverhältnis Alkohol zu Aldehyd sollte von 200:1 bis 1:1 gewählt werden. Die verwendete Katalysatormenge ist von untergeordneter Bedeutung. Sie kann im Bereich des 0,2 bis 10fachen des Gewichts an Aldehyd gewählt werden, wenn man diskontinuierlich arbeitet. Bei kontinuierlicher Arbeitsweise kann die Katalysatormenge das 100- bis 1-fache des Gewichts an je Stunde durch den Reaktionsraum geleiteten Aldehyds betragen.

Gegebenenfalls kann die Reaktion in einem Lösungsmittel, das gegen die Reaktionspartner inert ist, durchgeführt werden.

Als Sauerstoff setzt man molekularen Sauerstoff in reiner Form oder in Form eines Gemisches mit einem oder mehreren anderen Gasen, wie Stickstoff und Kohlendioxid, ein. So ist beispielsweise auch Luft geeignet. Es hat sich bewährt, den Sauerstoff in grösserer als für die Reaktion erforderlichen Menge einzusetzen. Vorzugsweise wird der Sauerstoff in wenigstens der 1,5fachen stöchiometrischen Menge verwendet.

Die Reaktion kann unter vermindertem, normalem oder erhöhtem Druck durchgeführt werden. Im allgemeinen bevorzugt man der Einfachheit halber normalen Druck.

Mit dem erfindungsgemässen Katalysator erzielt man schon nach kurzen Reaktionszeiten hohe Methacrolein-Umsätze von 65 bis 97% und ausgezeichnete Selektivitäten zu Methylmethacrylat von 85 bis 94%. Das aber führt zu einer beträchtlichen Erhöhung der Produktivität (definiert als g Methylmethacrylat pro g Pd pro Stunde) gegenüber den Katalysatoren, die aus dem oben erörterten Stand der Technik bekannt sind.

Beispiel 1

Herstellung von $Zn_6Al_2(OH)_{12}(CO_3)_3$ als Trägervorläufer

Zur Fällung des Katalysatorträgervorläufers werden 2 Lösungen benötigt:

Lösung 1:

54 Mole $Zn(NO_3)_2 \cdot 6\ H_2O = 16.065$ kg

und 18 Mole $Al(NO_3)_3 \cdot 9\ H_2O = 6.750$ kg

werden in Wasser gelöst und zu 36 l Lösung aufgefüllt, so dass eine 2-molare Lösung entsteht.

Lösung 2:

90 Mole technische Soda = 9.54 kg

werden in Wasser gelöst, so dass 45 l einer 2-molaren Lösung entstehen.

Beide Lösungen werden gleichzeitig, aber getrennt in einen Rührkessel gepumpt und bei 80 °C und einem pH-Wert von 7,0 vereinigt. Durch Steuerung der Lösungsmenge wird der pH-Wert während der Fällung konstant gehalten. Der als Fällung erhaltene Katalysatorträgervorläufer wird in einem nachgeschalteten Behälter bei 80 °C noch 15 bis 60 min nachgerührt. Der entstandene Niederschlag wird abfiltriert, nitratfrei gewaschen und sprühgetrocknet.

Die Entstehung der Verbindung $Zn_6Al_2(OH)_{12}(CO_3)_3$ kann an dem vorsichtig getrockneten Produkt röntgenographisch nachgewiesen werden. Sie ist durch folgende $Cu_{K\alpha}$-Linien im Guinier-Pulverdiagramm gekennzeichnet.

| d(Å) | 6.8 | 4.55 | 3.32 | 2.8 | 2.62 | 2.44 | 1.75 |
|---|---|---|---|---|---|---|---|
| Intensität | s. st. | s. st. | st. | m | st. | st. | m |

| 1.66 | 1.52 | 1.31 |
|---|---|---|
| st. | st. | m |

Für den Einsatz als Katalysatorträger ist besondere Vorsicht beim Trocknen nicht erforderlich, da ohnedies anschliessend calciniert und damit ein Gemisch aus ZnO und $Al_2O_3$ gebildet wird. Die Temperatur, bei der calciniert wird, hat Einfluss auf die innere Oberfläche des aus $Zn_6Al_2(OH)_{12}(CO_3)_3$ gebildeten Mischoxides. Bei 350 °C entsteht ein Träger mit 135 $m^2/g$, bei 650 °C ein solcher mit 74 $m^2/g$ BET-Oberfläche.

Beispiel 2

10 g $Zn_6Al_2(OH)_{12}(CO_3)_3$ werden bei 350 °C calciniert, mit 50 ml $H_2O$ versetzt und mit einer salzsauren Lösung von 0,33 g $PdCl_2$ unter Rühren ver-

mischt. Man rührt so lange, bis die überstehende Lösung farblos klar ist. Der Katalysator wird abgesaugt, mit 100 ml $H_2O$ in einen Kolben gegeben und unter Rühren 4 h bei 60 °C mit $H_2$ reduziert. Danach wird der Katalysator abgesaugt, mit einer Lösung von 0,33 g $Pb(OAc)_2 \cdot 3\ H_2O$ in 10 ml $H_2O$ vermengt, bei 60 bis 70 °C und 20 mbar getrocknet und unter $N_2$ abgekühlt. Der Katalysator enthält laut Analyse 1,7% Pd und 1,3% Pb und hat eine BET-Oberfläche von 44 $m^2/g$.

1,3 g des so dargestellten Katalysators füllt man in einen mit Thermostat auf 30 °C gehaltenen Glasreaktor (Schüttelente 250 ml), der mit einer Gasbürette, gefüllt mit Sauerstoff, über einen Schlauch verbunden ist, gibt eine Lösung von 1 g Methacrolein in 19 g Methanol zu, verschliesst die Schüttelente und bringt die Reaktion durch Schütteln des Glasreaktors in Gang. Nach 1 h entnimmt man eine Probe und analysiert gaschromatographisch. Man stellt fest, dass 97% des eingesetzten Methacroleins umgesetzt sind mit einer Selektivität von 85% zu Methylmethacrylat (MMA). Die Produktivität beträgt 50,3 m MMA/g Pd.h.

Beispiel 3

10 g $Zn_6Al_2(OH)_{12}(CO_3)_3$ werden bei 600 °C calciniert, mit 50 ml $H_2O$ versetzt und mit einer salzsauren Lösung von 0,33 g $PdCl_2$ unter Rühren vermischt. Man rührt so lange, bis die überstehende Lösung farblos klar ist. Der Katalysator wird abgesaugt, mit 100 ml $H_2O$ aufgeschlämmt und unter Rühren durch Zugabe von 3,12 ml einer 30%igen Formaldehyd-Lösung bei 60 °C während 2 h reduziert. Danach wird der Katalysator unter $N_2$ abgesaugt, mit einer Lösung von 0,33 g $Pb(OAc)_2 \cdot 3\ H_2O$ in 40 ml $H_2O$ vermengt, bei 60 bis 70 °C und 20 mbar Druck getrocknet und unter $N_2$ abgekühlt. Der Katalysator enthält laut Analyse 1,8% Pd und 1,4% Pb und hat eine BET-Oberfläche von 28 $m^2/g$.

4 g des so dargestellten Katalysators füllt man in einen mit Thermostat auf 40 °C gehaltenen Glasreaktor (Schüttelente, 1000 ml), der mit einer Gasbürette, gefüllt mit Sauerstoff, über einen Schlauch verbunden ist, gibt eine Lösung von 2 g Methacrolein in 48 g Methanol zu, verschliesst das Reaktionsgefäss und bringt die Reaktion durch Schütteln des Glasreaktors in Gang. Nach 0,5 h entnimmt man eine Probe und analysiert gaschromatographisch. Man stellt fest, dass 84,3% des eingesetzten Methacroleins umgesetzt sind mit einer Selektivität von 92,1% zu Methylmethacrylat (MMA). Die Produktivität beträgt 61,6 g MMA/g Pd.h.

Beispiel 4

Ein Katalysator wird hergestellt, indem man eine Mischung aus 4,5 g $TiO_2$-P25 (Degussa), 4,5 g ZnO (Merck), 1,0 g $La_2O_3$ und 0,2 g Graphit bei 600 °C 20 h tempert und anschliessend, wie im Beispiel 3 beschrieben, mit Pd und Pb belädt. Der Katalysator enthält laut Analyse 1,8% Pd und 1,4% Pb und hat eine BET-Oberfläche von 24 $m^2/g$.

Die Umsetzung von Methacrolein mit Sauerstoff und Methanol mit Hilfe dieses Katalysators erfolgt ebenfalls, wie im Beispiel 3 angegeben. Nach 1 h entnimmt man eine Probe und analysiert gaschromatographisch. Man stellt fest, dass 80,5% des eingesetzten Methacroleins umgesetzt sind mit einer Selektivität von 93,9% zu Methylmethacrylat. Die Produktivität beträgt 29,9 g MMA/g Pd.h.

Beispiel 5

10 g $Zn_6Al_2(OH)_{12}(CO_3)_3$ werden bei 700 °C calciniert und wie im Beispiel 3 beschrieben mit Pd und Pb belegt und reduziert. Der Katalysator enthält laut Analyse 1,7% Pd und 1,3% Pb und hat eine BET-Oberfläche von 17 $m^2/g$.

Die Umsetzung von Methacrolein und Sauerstoff an diesem Katalysator erfolgt ebenfalls wie in Beispiel 3 angegeben. Nach 0,5 h Versuchsdauer entnimmt man eine Probe und analysiert gaschromatographisch. Man stellt fest, dass 65% des eingesetzten Methacroleins umgesetzt sind mit einer Selektivität von 94,2% zu Methylmethacrylat. Die Produktivität beträgt 51,5 g MMA/g Pd.h.

Beispiel 6

Ein Katalysator wird hergestellt, indem man eine Mischung aus 4,5 g $TiO_2$-P25 (Degussa), 4,5 g ZnO (Merck) und 0,2 g Graphit bei 600 °C 20 h tempert und anschliessend wie in Beispiel 3 beschrieben, mit Pd und Pb belädt. Der Katalysator enthält laut Analyse 1,8% Pd und 1,4% Pb und hat eine BET-Oberfläche von 38 $m^2/g$.

Die Umsetzung von Methacrolein mit Methanol und Sauerstoff an diesem Katalysator erfolgt ebenfalls wie in Beispiel 3 angegeben. Nach 1 h Versuchsdauer entnimmt man eine Probe und analysiert gaschromatographisch. Man stellt fest, dass 67,5% des eingesetzten Methacroleins umgesetzt sind mit einer Selektivität von 89% zu Methylmethacrylat. Die Produktivität beträgt 23,5 g MMA/g Pd.h.

Beispiel 7

100 g $TiO_2$-P25 (Degussa) und 100 g ZnO (Merck) werden in 3 l $H_2O$ suspendiert und 12 h am Rückfluss erhitzt. Nach dem Erkalten wird der Katalysatorträger abgesaugt, zu Strängen mit 2 mm Ø verformt und 20 h bei 600 °C getempert. 100 g dieser Stränge werden mit 200 g einer wässrigen, salzsauren Lösung von $PdCl_2$, die 0,35% Pd enthält, in einem Glasrohr durch Umpumpen der Lösung so lange behandelt, bis die Lösung farblos wird (ca. 2 bis 4 h). Anschliessend gibt man zur Lösung noch 0,82 g $Pb(OAc)_2 \cdot 3\ H_2O$ und pumpt weitere 2 h um. Danach fügt man der Lösung 11,69 g einer 30%igen $CH_2O$-Lösung zu und reduziert den Katalysator bei 60 °C 4 h. Der Katalysator enthält laut Analyse 0,52% Pd und 0,44% Pb und hat eine BET-Oberfläche von 15 $m^2/g$.

100 g dieses Katalysators füllt man in ein mit Thermostat versehenes Glasrohr von 1 m Länge und 1,1 cm Durchmesser als Reaktor.

Vom unteren Ende des bei 40 °C gehaltenen Reaktors werden stündlich 2 l Sauerstoff und vom oberen Ende 10 g einer 10%igen Lösung von

Methacrolein in Methanol eingeführt. Die Reaktionslösung wird mit einer Frequenz von 2 l/h im Kreis gepumpt, so dass die Reaktion in einem kontinuierlichen Strömungssystem durchgeführt wird. Nach 48 h beträgt der Umsatz an Methacrolein 84,5%, die Selektivität zu Methylmethacrylat 83,7%; nach 300 h beträgt der Umsatz 83,5%, die Selektivität 85,9%. Die Produktivität beträgt 2,0 g MMA/g Pd.h.

Beispiel 8

200 g $Zn_6Al_2(OH)_{12}(CO_3)_3$ werden zu Strängen von 3 mm Durchmesser verarbeitet und anschliessend 6 h bei 350 °C calciniert. 100 g dieser Stränge werden mit einer salzsauren Lösung von 3,3 g $PdCl_2$ in 500 ml $H_2O$ durch gelegentliches Umrühren so lange behandelt, bis die überstehende Lösung farblos klar ist. Das Wasser wird abdekantiert und der zurückbleibende Katalysator anschliessend mit 500 ml einer 5%igen Formaldehyd-Lösung 16 h bei 60 °C reduziert. Die Lösung wird entfernt und der Katalysator mit einer Lösung von 3,3 g $Pb(OAc)_2 \cdot 3\,H_2O$ in 300 ml $H_2O$ 4 h behandelt. Der Katalysator wird anschliessend bei 60 °C unter $N_2$ getrocknet. Der Katalysator enthält laut Analyse 1,3% Pd und 0,85% Pb und hat eine BET-Oberfläche von 24 $m^2$/g.

26 g dieses Katalysators füllt man in ein mit Thermostat versehenes Reaktorrohr von 0,3 m Länge und 1,1 cm Durchmesser. Vom unteren Ende des bei 40 °C gehaltenen Reaktorrohrs werden stündlich 16 g einer 10%igen Lösung von Methacrolein in Methanol und 1,3 l Sauerstoff eingeführt. Nach 7 h beträgt der Umsatz an Methacrolein 79,9%, die Selektivität zu Methylmethacrylat 92,2%; nach 300 h beträgt der Umsatz 78,9%, die Selektivität 89,7%. Die Produktivität beträgt 4,8 g MMA/g Pd.h.

Beispiel 9

Ein Katalysator wird hergestellt, indem man eine Mischung aus 6 g $La_2O_3$, 4 g $TiO_2$-P25 (Degussa) und 0,2 g Graphit bei 600 °C 20 h tempert und anschliessend, wie im Beispiel 3 beschrieben, mit Pd und Pb belädt. Der Katalysator enthält laut Analyse 1,8% Pd und 1,2% Pb und hat eine BET-Oberfläche von 13 $m^2$/g.

Die Umsetzung von Methacrolein mit Methanol und Sauerstoff an diesem Katalysator erfolgt ebenfalls, wie im Beispiel 3 angegeben. Nach 1 h entnimmt man eine Probe und analysiert gaschromatographisch. Man stellt fest, dass 65% des eingesetzten Methacroleins mit einer Selektivität von 91% zu Methylmethacrylat umgesetzt sind. Die Produktivität beträgt 23,5 g MMA/g Pd.h.

**Patentansprüche**

1. Katalysator für die Herstellung con Carbonsäureestern aus Aldehyden und Alkoholen in Gegenwart von Sauerstoff, der als aktive Bestandteile Palladium und Blei enthält, dadurch gekennzeichnet, dass

a) die aktiven Bestandteile sich auf einem Träger befinden, der wenigstens zwei der Oxide ZnO, $Al_2O_3$, $La_2O_3$ und $TiO_2$ enthält, wobei $Al_2O_3$ und $TiO_2$ als alleinige Bestandteile ausgeschlossen sind, und

b) der Katalysator nicht mehr als 0.02% an Alkali oder Erdalkali, berechnet als Metall, enthält.

2. Katalysator nach Anspruch 1, dadurch gekennzeichnet, dass die aktiven Bestandteile sich auf einem Träger befinden, der durch Calcinieren der Verbindung $Zn_6Al_2(OH)_{12}(CO_3)_3$ entstanden ist.

3. Verwendung eines Katalysators nach Anspruch 1 oder 2 zur Herstellung von Methylmethacrylat aus Methacrolein, Methanol und Sauerstoff bei Temperaturen von 20 bis 100 °C.

4. Verwendung gemäss Anspruch 3 in flüssiger Phase.

**Claims**

1. A catalyst, containing palladium and lead as active constituents, for the preparation of carboxylic acid esters from aldehydes and alcohols in the presence of oxygen, wherein

(a) the active constituents are supported on a carrier which contains at least two of the oxides ZnO, $Al_2O_3$, $La_2O_3$ and $TiO_2$, $Al_2O_3$ and $TiO_2$ as the only constituents being excluded, and

(b) the catalyst contains not more than 0.02% of alkali or alkaline earth, calculated as metal.

2. A catalyst as claimed in claim 1, wherein the active constituents are supported on a carrier formed by calcining the compound $Zn_6Al_2(OH)_{12}(CO_3)_3$.

3. The use of a catalyst as claimed in claim 1 or 2 for the preparation of methyl methacrylate from methacrolein, methanol and oxygen at from 20 to 100 °C.

4. The use as claimed in claim 3 in the liquid phase.

**Revendications**

1. Catalyseur pour la préparation d'esters d'acide carboxylique à partir d'aldéhydes et d'alcools en présence d'oxygène, contenant du palladium et du plomb en tant que composants actifs, caractérisé en ce que

a) les composants activs se trouvent sur un support qui contient au moins deux des oxydes ZnO, $Al_2O_3$, $La_2O_3$ et $TiO_2$, $Al_2O_3$ et $TiO_2$ étant exclus en tant que composants uniques et

b) le catalyseur ne contient pas plus que 0,02% de composés alcalins ou alcalinoterreux, exprimés en métal.

2. Catalyseur selon la revendication 1, caractérisé en ce que les composants actifs se trouvent sur un support qui a été formé par calcination du composé $Zn_6Al_2(OH)_{12}(CO_3)_3$.

3. Utilisation d'un catalyseur selon la revendication 1 ou 2 pour la préparation de méthacrylate de méthyle à partir de méthacroléine, de méthanol et d'oxygène à des températures de 20 à 100 °C.

4. Utilisation selon la revendication 3 en phase liquide.